# EUROPEAN PATENT SPECIFICATION

(11) **EP 2 519 154 B1**
(45) Date of publication and mention of the grant of the patent: **25.01.2017**
(21) Application number: 10841376.6
(22) Date of filing: 22.12.2010
(51) Int. Cl.: A61B 5/145, A61B 5/1486, C12Q 1/00, G01N 33/48

(54) **SENSOR ARRANGEMENT FOR CONTINUOUSLY MEASURING ANALYTES IN A BIOLOGICAL FLUID**
SENSORANORDNUNG ZUR KONTINUIERLICHEN ANALYTMESSUNG IN EINER BIOLOGISCHEN FLÜSSIGKEIT
AGENCEMENT DE CAPTEUR POUR LA MESURE EN CONTINU D'ANALYTES DANS UN FLUIDE BIOLOGIQUE

(30) Priority: 30.12.2009 SE 0951038; 30.12.2009 US 291241 P
(43) Date of publication of application: 07.11.2012
(73) Proprietor: Maquet Critical Care AB, 171 54 Solna (SE)
(72) Inventor: KARLSSON, Anton, S-122 66 Enskede (SE); CARLSSON, Anders, S-752 28 Uppsala (SE); JOBST, Gerhard, 79356 Eichstetten (DE)
(74) Representative: Bergenstråhle Group AB
(86) International application number: PCT/SE2010/051456
(87) International publication number: WO 2011/081596

(56) References cited:
- EP-A1- 0 539 625
- WO-A1-2010/002350
- US-A- 5 120 420
- US-A- 6 013 029
- US-A1- 2002 072 103
- US-A1- 2003 023 189
- US-A1- 2003 214 304
- US-A1- 2004 082 804
- US-A1- 2009 143 658
- MIN ZHAO ET AL: "An oxygen-rich fill-and-flow channel biosensor", BIOSENSORS & BIOELECTRONICS ELSEVIER UK, vol. 18, no. 5-6, May 2003 (2003-05), pages 827-833, XP002689232, ISSN: 0956-5663

## Description

### FIELD OF INVENTION

The present invention relates to a sensor for continuously measuring at least one analyte in a liquid flow with improved reliability.

### BACKGROUND OF THE INVENTION

Since recently it is known that certain substances that may be present in the body can function as indicators for various pathological conditions. Such substances are hereafter called indicator substances or analytes. Examples of indicator substances are glucose, lactate, pyruvate, glycerol, glutamate, and glutamine and heart specific enzymes. Pathological conditions that may be indicated or detected, or as well forecasted, include ischemia, hypoglycemia, hyperglycemia, sepsis, cell membrane damage or lipolysis, vasospasms and metabolic disorders. By measuring indicator substances, pathological conditions may be detected before they lead to clinical signs. It may even be possible to detect processes or conditions that eventually may lead to a pathological condition. In many cases it would be advantageous to have the possibility to measure the concentration of indicator substances directly in a blood stream, or in tissue fluid. However, until now there have not existed any systems suitable for clinical use for measuring indicator substances. Systems known from the background art all have different drawbacks. Examples of common drawbacks in background art systems are that the measurement delay is extensive and that one has measured phenomena that are the result of a pathological condition, e.g. ischemia. This is clearly disadvantageous. With measurement delay is meant the time that passes from the moment that a sample is taken until the moment that a measurement value relating to this sample is obtained. In background art systems also measurement values can often only be obtained with relatively extended time periods, between each measurement value, e.g. if sample fluid is collected in micro vials. Faced with the aim or task to develop a reliable and accurate measuring system that can be used in monitoring the condition of a subject, e.g. a patient, in a critical condition or situation, the skilled person is faced with other problems and situations than those which previously have aroused. For this purpose more or less continuously analyte measuring sensors have been developed especially for measuring glucose. However, such sensors often are quite unreliable, a situation which may be aggravated when the sensors have multiple functions such as simultaneous measurement of several analytes. Sensors including electrochemical electrodes for continuously measuring several analytes can be unreliable due to interfering processes or reactions and variations of components participating in the electrochemical reactions. A number of techniques to circumvent or to safe-guard from such problems have developed. US Patents 7,074,307 and 7,108,778 serve as examples of certain solutions to problems in accurate continuous sensing of anklets in body fluids. US Patent Application 2003/0214304 describes a sensor for continuous monitoring of biological sample for e.g. glucose which is adapted to determine the sample flow rate. For this purpose the sensor is arranged with a first and second measuring electrode for determining the sample flow rate from their responses. To meet this requirement both measuring electrodes are exemplifies to have similar performances and this arrangement has no provisions for handling disturbing side-products generated from the first electrode. It is evident that there are a number of technical problems yet to overcome when designing multifunctional continuously measuring sensors to reach even higher reliability and safety in clinical applications for measuring substances in a body fluid.

The present invention aims at providing a sensor for continuous measurement of analytes in a flowing liquid that has an improved reliability and capacity to counteract measuring errors, thereby making the sensor especially suitable for inclusion into an arrangement to survey intensive care patients, who benefit from rigid control of the concentration of certain compounds (indicator substances) in the blood.

### DESCRIPTION OF THE INVENTION

Generally, the invention relates to a sensor for continuous detection of one or more analytes in a liquid flow. The sensor generally comprises an electrode part and a flow distributor for leading the liquid flow with the analyte(s) to the electrode part in order to enable a correct sensing function. The electrode part comprises an array of electrodes together forming an essentially planar sensing surface generally forming an upper surface facing the liquid flow. The flow distributor comprises a flow inlet, a flow channel and a flow outlet in order to establish a liquid flow of analytes along the sensing surface.
It is an important part of the invention that the flow distributor and the array of electrodes are configured to counteract problems that can envisaged to disturb the sensor to correctly determining the concentration of the analytes. These problems include, but are not limited to, formation of air bubbles, interfering agents, and crosstalk between the electrodes. For this reason, the flow distributor comprises a ceiling having a surface facing said flow channel that is more hydrophobic than said sensing surface, flow inlet and the flow outlet are located in the plane of said ceiling surface different to the plane, but essentially parallel to that, of the sensing surface. Furthermore, the array of electrodes is arranged so that, in the direction from the flow inlet to the flow outlet, the array of electrodes consecutively comprises a first blank electrode, at least a first measuring electrode, a second blank electrode, at least a second measuring electrode and optionally a third blank electrode. The first and second electrode can be configured to measure the same or different analytes.

The measuring electrodes preferably comprise an enzyme capable of enzymatically converting the selected analyte and thereby providing a signal representative of the concentration of said analyte. The blank electrodes preferably are arranged identical to the measuring electrodes, but comprise no enzyme. The blank electrodes are capable of detecting and quantifying the level of interference (such as chemical interference and electrical disturbances) that can distort results from the measuring electrodes.
In one embodiment of the sensor, the at least one measuring electrode is a glucose electrode, or a lactate electrode, or a pyruvate electrode.
In one embodiment of the sensor, the at least one measuring electrode is a glucose electrode and a lactate electrode.
In one embodiment of the sensor, the at least one measuring electrode is a lactate electrode and a pyruvate electrode.
In one embodiment of the sensor the at least one measuring electrode is a glucose electrode and a pyruvate electrode.
In one embodiment of the sensor, the at least one electrode is a glucose electrode, a lactate electrode and a pyruvate electrode.
In one embodiment, the sensor comprises an electrode array consecutively comprising a first blank electrode, a first glucose electrode, a second blank electrode, a second glucose electrode and optionally third blank and glucose electrodes. Alternatively, in a preferred embodiment the sensor comprises an electrode array consecutive comprising prises a first blank electrode a first glucose electrode, a first lactate electrode, a second blank electrode, a second glucose electrode and a second lactate electrode. In one embodiment of the sensor, the array of electrodes is arranged so that, in the direction from the flow inlet to the flow outlet, it consecutively comprises a first blank electrode, a first lactate electrode, a first glucose electrode, a second blank electrode, a second lactate electrode and a second glucose electrode. This embodiment can be further modified so it comprises a first pyruvate electrode located between the first blank and the second blank electrode, and a second pyruvate electrode located after the second blank electrode.
In an embodiment when the sensor comprises at least two glucose or lactate or pyruvate electrodes, the at least two electrodes that are directed to measure the same analyte can suitably have different intervals of linear sensitivity, i.e. when the response of the electrodes assumes linearity to the concentration of analyte. For example, a sensor with glucose sensing capacity is arranged with several glucose electrodes with different intervals of linear sensitivity. For example, an array of electrodes can consecutively comprise a blank electrode, a first glucose electrode having a first interval of linear sensitivity, a second blank electrode, a second glucose electrode having a second interval of linear sensitivity, and optionally a third blank electrode and a third glucose electrode having a third interval of linear sensitivity. In a suitable arrangement the first interval of linear sensitivity relates to the highest concentration of glucose. For example, the first interval of linear sensitivity corresponds to glucose concentrations between about 5 to 100 mM, the second interval of linear sensitivity corresponds to glucose concentrations between about 1 to 25 mM, and the third interval of linear sensitivity corresponds to glucose concentrations between about 0.1 to 5 mM. Alternatively, first and second lactate and pyruvate electrodes can have different intervals of linear sensitivity. For example a sensor with first and second glucose, lactate and pyruvate electrodes can have different linear sensitivities between first set and second sets electrodes. The sensor according further suitably comprises a reference electrode and/or a counter electrode. The reference electrode and the counter electrode have ordinary functions as expected by person skilled in the art. In the sensor, the reference electrode and/or a counter electrode is/are located after the measuring and blank electrodes, for example to reduce the risk that any product they produce interferes with the measurements
The sensor is adapted to low liquid flows and accordingly flow channels of small dimensions. As previously described, the flow distributor has a ceiling part constituingthe upper surface of a flow channel along the oppositely located sensing surface. The flow inlet and the flow outlet are located in the plane of the ceiling surface , which is essentially parallel to the plane represented by the sensing surface. Accordingly, the flow inlet and outlet are provided in an upper part of the sensor and a liquid flow of analytes is established in the flow channel along the sensing surface. As also described, in order to counteract entrapment of air bubbles at the sensing surface, the ceiling part is made of a material that is more hydrophobic than the sensing surface. In one example the ceiling surface made from PMMA (poly(methyl methacrylate) and the sensing surface comprises at least partially a hydrophilic hydrogel. It is also conceivable to employ other hydrophobic polymeric materials like brands of PTFE (polytetrafluoroethene) and suitable brands of polystyrene (PS). The first upper membrane of the electrodes of the sensor can comprise the hydrophilic hydrogel in order to provide the sensing surface with a required hydrophilicity. In suitable embodiments where the electrodes comprise hydrogen peroxide generating oxidases, the first upper membrane comprises catalase, as will be explained in following sections. It is also conceivable and part of the present invention to cover the entire sensing surface with such a hydrophilic hydrogel. In general terms, the flow distributor is designed to admit a fluid flow of about 0.1 to 50 microliters per minute in its flow channel. Other dimensions are defined in following sections. The sensor includes at least one measuring electrode with multiple membrane layers. The layers comprise an oxidase membrane layer with immobilized oxidase enzyme, such as glucose, lactate oxidase or pyruvate oxidase capable of reacting the analyte with oxygen in a hydrogen peroxide generating reaction; and a diffusion limiting membrane adapted to provide a higher diffusion resistance for the analyte than for oxygen and to provide a lower flow of analyte to the oxidase membrane layer than the conversion rate of the oxidase enzyme. In a preferred embodiment the diffusion limiting membrane has a thickness of about 10 micrometer. Preferably, the diffusion limiting membrane is made from a hydrogel, preferably the hydrogel is poly-HEMA. The oxidase membrane layer has an area adapted so that the output signal of said measuring electrode is sufficiently high in relation to a potential noise level or noise signal for the lowest analyte concentration in the linear measurement range of the measuring electrode. Preferably, the oxidase membrane layer has an essentially circular area with a diameter from about 250 to about 1000 micrometer, preferably about 450 micrometer. The measurement electrodes can further comprise a membrane with selective permeability, for example to limit or exclude other reactive agents than hydrogen peroxide from reaching the platinum anode of the electrode.
The sensor further preferably comprises a catalase membrane with a sufficient extension and catalase activity to substantially decompose all the hydrogen peroxide reaching the membrane. Preferably, the catalase membrane has a thickness in the interval of 5 to 10 micrometer. A catalase membrane supports inclusion of several oxidase containing electrodes in the sensor by reducing risk of disturbances from migrating hydrogen peroxide. Any such "cross talk" between the electrodes can also be detected by and quantified by the purposefully arranged blank electrodes. Thus, using the measured signal from the blank electrodes the response for the down-stream electrodes can be corrected for possible cross talk, resulting in more accurate measurement of the analyte(s).
According to the invention, the sensor as described above and in its various general and specific embodiments is especially useful for continuous measurement of one or more of glucose, lactose and pyruvate, especially in clinical intensive care applications when high reliability is desired. The arrangement with several measurement electrodes for the same analyte and several blank electrodes admits that signals can be compared and processed with algorithms to balance out errors from interfering events (interfering active agents, air bubbles, cross talk, electrical disturbances etc) and to obtain more accurate measurements. In conclusion the sensor when used to monitor a patient in a clinical situation provides an increase in safety.

The term "analyte" as used herein is a broad term and is used in its ordinary sense, including, without limitation, to refer to a substance or chemical constituent in a biological fluid (for example, blood, interstitial fluid, cerebral spinal fluid, lymph fluid or urine) that can be analyzed. Analytes can include naturally occurring substances, artificial substances, metabolites, and/or reaction products. The term" liquid flow" as used herein represent a flow of liquid medium carrying the analyte and can for example be a perfusion fluid (dialysate) carrying the analytes from a point of interaction with a body or it can be blood.

The term "electrode" as used herein is a broad term, and is to be given its ordinary and customary meaning to a person of ordinary skill in the art (and it is not to be limited to a special or customized meaning), and refers without limitation to a conductor through which electricity enters or leaves something such as a battery or a piece of electrical equipment. In one embodiment, the electrodes are the metallic portions of a sensor (e.g., electrochemically reactive surfaces) that senses the products of the analyte reactions. In some embodiments, the term electrode includes the conductive wires or traces that electrically connect the electrochemically reactive surface to connectors (for connecting the sensor to electronics) or to the electronics.

The term "sensing surface" shall represent the collective surface of the electrodes that contacts the liquid flow of analytes.

The term "enzyme" as used herein is a broad term, and is to be given its ordinary and customary meaning to a person of ordinary skill in the art (and it is not to be limited to a special or customized meaning), and refers without limitation to a protein or protein-based molecule that speeds up a chemical reaction occurring in a living being. Enzymes may act as catalysts for a single reaction, converting a reactant (also called an analyte herein) into a specific product. In one exemplary embodiment of a glucose oxidase-based glucose sensor, an enzyme, glucose oxidase (GOX) is provided to react with glucose (the analyte) and oxygen to form hydrogen peroxide.

Continuous monitoring is to be understood as monitoring with presentation of a data value with an interval shorter than 10 min, however in some embodiments the interval is shorter than 5 minutes and in some embodiments the interval is shorter than 1 minute and in some embodiments the interval is shorter than 10 seconds and in yet other embodiments the interval is shorter than 2 seconds. Shorter intervals provide more data. The more data available the more mean calculation, transformation and filtering are possible, which creates output data from the sensor that is less affected by disturbances and thus more accurate.

### DETAILED DESCRIPTION OF THE INVENTION

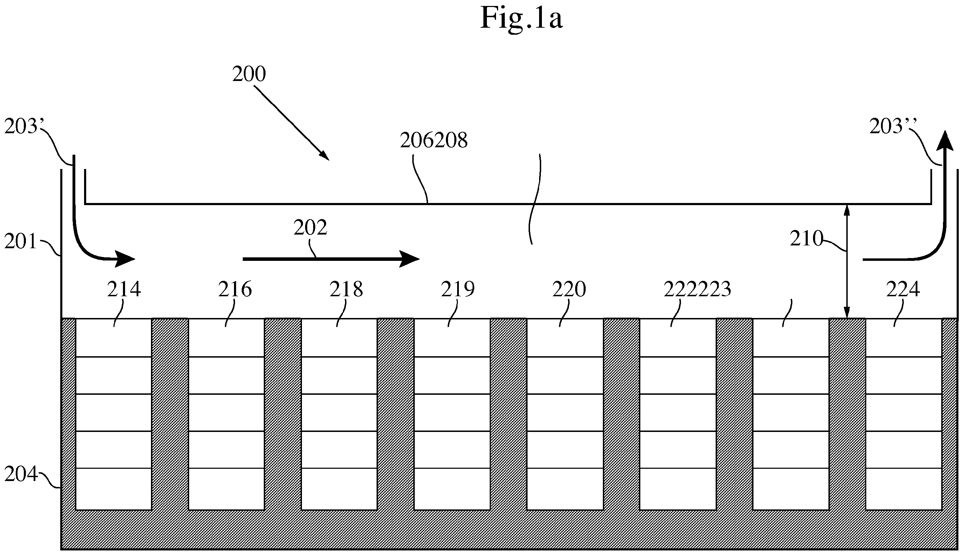

Figs. 1a- shows schematically an embodiment of the sensor 200.

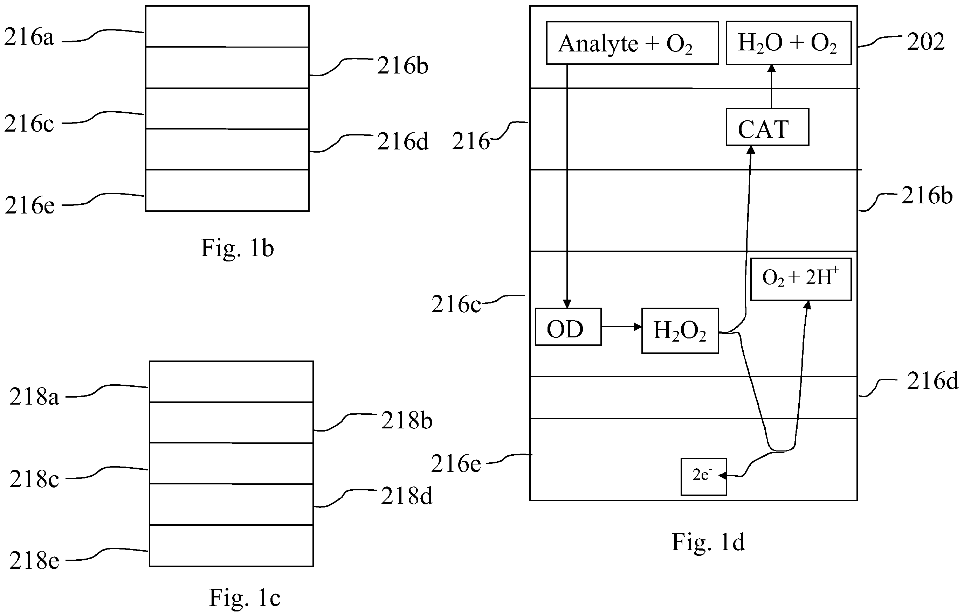

Figs. 1b and 1c are drawings schematically showing detailed views of the sensor electrodes 216 and 218 that can be used in the sensor of Fig. 1a.
Fig. 1d gives a schematic view of the main reaction and transport pathways of a measuring electrode in the sensor 200.

Before the system described herein is described in detail, it is to be understood that this system is not limited to the particular component parts of the devices described or steps of the methods described as such devices and methods may vary. It is also to be understood that the terminology used herein is for purposes of describing particular embodiments only, and is not intended to be limiting. It must be noted that, as used in the specification and the appended claims, the singular forms "a," "an" and "the" also include plural referents unless the context clearly dictates otherwise. Thus, for example, reference to "an element" includes more than one such element, and the like.

In the embodiment outlined in Fig. 1a the sensor generally comprises an electrode part 204 comprising the electrode array 214 to 224 and a flow distributor 201. The electrode array consecutively includes, a first blank electrode 214, a first pyruvate electrode 216, a first lactate electrode 218, a first glucose electrode 219, a second blank electrode 220, a second pyruvate electrode 222, a second lactate electrode 223 and a second glucose electrode 224. The detailed functionality and arrangements of the measuring electrodes and the blank electrodes will be outlined below in the context of Figs. 1b to 1d. The array of electrodes 214-224 together form an essentially planar sensing surface in the flow channel 208 having a height indicated 210.

The flow distributor 201 of the upper part of sensor has a ceiling part 206 constituting a ceiling surface that provided with an inlet 201 and an outlet 201' for a liquid flow that contains among other substances the analytes, here glucose, lactate and pyruvate; and oxygen (02) and arrives from a sampling station upstream the sensor. The ceiling part is made from PMMA in order to become more hydrophobic than the opposite sensing surface. In order to allow for controlled flowing conditions of the flow 202 in the flow channel, also in shallow flow channels 20, the inlet 201 is arranged in a plane above, but essentially parallel to the sensing surface. The liquid flow can be the dialysate from a microdialysis or ultrafiltration equipment or any other equipment capable of interacting with a body fluid or tissue to pick up a liquid flow of analytes in a sampling station. The liquid flow can also be whole blood as sampled with a probing instrument. The sensor as arranged in Fig. 1a and in other embodiments described herein can be a part of an invasive instrument, be implanted in the patient or be placed in an extracorporeal position. It is also contemplated, but not a particular part of the present invention, that signals generated by the sensor shall be transmitted (electrically or wirelessly) and processed to a monitoring function of a suitably function for surveying intensive care patients or other patients requiring continuous surveillance.

With reference to Figs. 1b, 1c and 1d the design and function of the measuring electrodes will be described more in detail. Short description of the different membranes/layers in the measuring electrode 216:
216a: Catalase membrane
216b: Enzyme-free diffusion limiting membrane
21 6c: Oxidase membrane, here lactate oxidase membrane
216d: Selectively permeable membrane
216e: Platinum anode
Now turning to Fig. 1b for explaining the arrangements of a lactate electrode 216 (similarly arranged as lactate electrodes 218,223 in Fig. 1a, also in Fig. 1a the electrode signified 216 is a pyruvate electrode). In the oxidase membrane 216c a reduction/oxidation (redox) process takes place involving the analyte and the oxygen. In this redox process the analyte is oxidized and the oxygen is reduced. The products of this process are hydrogen peroxide and the oxidation product of the analyte. The oxidation product of the analyte diffuses out to the liquid flow 202 and is washed away with the flow 202. A part of the hydrogen peroxide diffuses upwards in the measuring electrode 216 and another part diffuses towards the platinum anode 21 6e. The layer 216c is in this case a lactate oxidase membrane since the measuring electrode 216 is measuring lactate. This layer is a membrane in which the enzyme lactate oxidase is immobilized, preferably the membrane is a poly-HEMA-hydrogel membrane (poly-HEMA=Poly 2-Hydroxyethylmethacrylate). In the oxidase membrane 216c the immobilized enzyme lactate oxidase acts as a catalyst when the lactate that reaches the oxidase membrane 21 6c reacts with oxygen and hydrogen peroxide is produced. Some of the hydrogen peroxide that is produced diffuses upwards in the direction of the enzyme-free diffusion limiting membrane 216b and the catalase membrane 216a. When this hydrogen peroxide reaches the catalase membrane 216a it is decomposed by the catalase membrane 21 6a into oxygen and water. The two membranes diffusion limiting membrane 216b and catalase membrane 21 6a are described more in detail below. The layer 216d is a selective membrane that only, or at least substantially only, is permeable to hydrogen peroxide. Advantageously the layer 216d is an electropolymerized permselective membrane. The selective membrane 216d is advantageous since it suppresses electrochemical interference, otherwise there would be a risk that other substances, than hydrogen peroxide, could reach the platinum anode 216e and give rise to erroneous readings regarding the concentration of lactate in the liquid flow 202. The hydrogen peroxide penetrates through the selective membrane 216d and is oxidised to oxygen at the platinum anode 21 6e. The oxidation of the hydrogen peroxide is achieved since the platinum anode 216e is polarized at a potential, versus the reference electrode, where electro-oxidation of hydrogen peroxide occurs.

Hence, at the platinum anode 216e the hydrogen peroxide is detected and the amount of hydrogen peroxide detected is proportional to the amount of lactate present in the liquid flow 202. Depending on the amount of hydrogen peroxide reaching the platinum anode 21 6e within a certain time period, different amounts of electrons per time period is produced, and hence gives different levels of the output signal.

The layer 216b is an enzyme-free diffusion limiting membrane, advantageously a poly-HEMA-membrane, for controlling the diffusion of the analyte, e.g. lactate. The diffusion limiting membrane 216b controls how quickly the lactate, or how much lactate per time-period that, reaches the oxidase membrane 216c. In the dialysate 202 the concentration of oxygen is much lower than the concentration of the analyte. One common situation is to have a concentration of 5 to 10 mmol/l of the analyte, e.g. lactate, and a concentration of 0.2 mmol/l of oxygen. If this difference in concentration would be present in the oxidase membrane 216c, there would not be enough oxygen present for the redox process in the oxidase membrane. Therefore the diffusion limiting membrane 216b suitably reduces the diffusion speed or rate for oxygen to be 3 to 5 times lower than without the membrane 216b and suitably reduces the diffusion rate for the analyte, e.g. lactate or glucose, to be around 1000 times lower than without the membrane 21 6b. The reason why the diffusion limiting membrane 216b can hinder the diffusion of the analyte much stronger than the diffusion of the oxygen is that the oxygen molecules are much smaller than the molecules of the analyte. By choosing an appropriate material and thickness of the diffusion limiting membrane 216b, the above mentioned difference in limitation of diffusion rate can be achieved. Because of this difference in reducing diffusion rate, the diffusion limiting membrane 216b brings the positive effect that the concentrations of oxygen and analyte is more in balance after the diffusion limiting membrane 216b, i.e. in the oxidase membrane 216c, which is desirable since it can be ensured that there is sufficient, or a surplus of, oxygen present for the redox process in the oxidase membrane 216c.

A sensor can have several measuring electrodes for each measured substance, e.g. 2 or 3 measuring electrodes for lactate. In this way each measuring electrode can be optimized for a certain interval of the concentration of the analyte (e.g. glucose, lactate, pyruvate, glycerol, glutamate or glutamine) in the liquid flow. A higher thickness or density of the enzyme-free diffusion limiting membrane 216b makes it possible to measure higher concentrations of a substance or analyte present in the liquid flow but to measure low concentrations of a substance, the thickness or density of the enzyme-free diffusion limiting membrane 216b must not be too high so that the measuring electrode has the sensitivity necessary to obtain reliable measurements also for low concentrations of a substance present in the liquid flow.

The catalase membrane 216a prevents hydrogen peroxide from diffusing upwards from the oxidase membrane 216c from reaching the liquid flow 202 and in this way prevents cross-talk between the different measuring electrodes. Hydrogen peroxide that reaches the catalase membrane 216a from the oxidase membrane 216c is decomposed within the catalase membrane 216a. The catalase membrane 216a also brings an extremely low flow rate dependency because hydrogen peroxide that otherwise would accumulate within the dialysate 202 is decomposed in the catalase membrane 21 6a. The very low flow rate dependency is advantageous in achieving a high accuracy. If hydrogen peroxide would accumulate within the liquid flow 202, this would lead to an increase in the sensor signal measured at the platinum anode 216e. This is a problem in measuring electrodes having no catalase membrane 216a covering the oxidase membrane 216c. The flow rate dependency in those measuring electrodes makes it difficult to obtain a measuring electrode with high accuracy. If there would be no catalase membrane 216a hydrogen peroxide would accumulate in the liquid flow 202 above the measuring electrode 216 and would, at least partially, diffuse down through the measuring electrode 216 and increase the sensor signal. How much of the hydrogen peroxide accumulated in the liquid fluid 202 that would diffuse down through the measuring electrode 216 would be dependent on the flow rate of the liquid flow 202. Hence, the output signal of the measuring electrode would be dependent on the flow rate of the liquid flow 202.

In the preferred embodiment outlined in Fig. 1a the sensor consecutively includes a first blank electrode 214, a first pyruvate electrode 216, a first lactate electrode 218, a first glucose electrode 219, a second blank electrode 220, a second pyruvate electrode 222, a second lactate electrode 223 and a second glucose electrode 224 (the electrodes are arranged as described in the foregoing section of the description). The first glucose electrode 219 has a design similar to the first lactate electrode 218, the second lactate electrode 223 and the second glucose electrode 224 have the same design and function as the first electrodes 218 and 219 with their respective oxidases present in corresponding oxidase membranes, while including same membrane arrangements as the first lactate electrode 218. Also the first and second pyruvate electrode 216 and 222 are arranged in accordance with the first lactate electrode 218, but having pyruvate oxidase present in the oxidase membrane,.

It is also necessary to consider that a supply of phosphate is necessary for converting pyruvate in the detecting reaction. Alternative electrode designs are also conceivable, for example where measuring electrodes for the same analyte but having different linear ranges. The sensor outlined in Fig. 1 a further comprises a reference electrode and a counter electrode (not depicted) with conventional functionality and outline. These electrodes can be located after the second blank electrode in order to minimize any interference from them on the measuring electrodes.

The blank electrodes 214 and 220 have a design similar to the measuring electrodes but are free from enzyme in layers 214c, 220c. In these layers there is only the membrane material, e.g. a hydrogel membrane, present wherein the immobilized enzymes are kept in the measuring electrodes. One reason for providing the first blank electrode 214 is to detect any hydrogen peroxide, or other electroactive substances, e.g. ascorbic acid or paracetamol, present in the liquid flow 202 already before the liquid flow 202 arrives to the measuring electrodes, in order to establish a reference level for the signals obtained from the measuring electrodes. If the output signal from the first blank electrode 214 would be very high that may be a sign of an error in the system and the output signals from the measuring electrodes obtained at that point of time can be discarded, if appropriate.

By providing two electrodes each for lactate and glucose, redundancy is achieved and the reliability and accuracy of the system 100 is improved since if a fault arises in one measuring electrode, the other can still be used. It is more unlikely that two measuring electrodes should be erroneous than that an error occurs in one measuring electrode. By comparing the readings or sensor signals from two measuring electrodes measuring the same substance it can be determined if the measuring electrodes function correctly, or if one of them gives an erroneous reading. The possibility to detect such erroneous readings increases the accuracy of the system 100 since the probability to have access to a sensor signal from a properly functioning measuring electrode is increased. A further advantage of having more than one sensing electrode is that the signals from the sensing electrodes can be averaged, thus reducing the possible variation between electrodes, leading to more accurate measurements.

One reason for providing the second blank electrode 220 is to detect any potential cross talk between the measuring electrodes. That is, e.g. to detect potential hydrogen peroxide present in the liquid flow in the flow channel 208. If for example the catalase membrane of one of the first measuring electrodes would not function properly hydrogen peroxide from that measuring electrode could enter into the flow channel 208. Such a situation can be detected by comparing the signals from the first blank electrode 214 and the second blank electrode 220. Thus, using the measured signal difference from the blank electrodes the response for the down-stream electrodes can be corrected for possible cross talk, resulting in more accurate measurement of the analyte(s). Increased signals from (any of) the blank electrodes will indicate an accumulation of peroxide in the sensor and thereby be indicative of an insufficient or incorrect flow through the sensor.

One advantageous measure for the flow channel 208 is a flow channel height 210 of approximately 75 micrometer and a flow channel width 211 of approximately 450 micrometer. A suitable interval for the flow channel width 211 is 250 to 1000 micro meters. A flow channel width 211 of 250 micrometer is a suitable lower limit since that width still renders the area of the oxidase membrane 216c sufficiently large. With a smaller flow channel width 211 than 250 micrometer problems may be encountered with a too low signal level from the sensor, because a too small area of the oxidase membrane 216c can result in a too small production of hydrogen peroxide in the oxidase membrane. This depends on the lowest analyte concentration that the measuring electrode should be able to detect with sufficient accuracy. The oxidase membrane 216c may have a circular or essentially circular shape, as seen in the direction of the arrows at "A" in Fig. 2a. In this case a suitable interval for the dimensions of the oxidase membrane is a diameter of 250-1000 micrometer, suitably 250-700 micrometer, most preferably about 450 micrometer. A flow channel width of 1000 micrometer is a suitable upper limit to limit the internal volume in the system to advantageously limit the delay in the system.

## Claims

1. A sensor for continuous detection of one or more analytes in a liquid flow, comprising:
an electrode part 204 comprising an array of electrodes 214-224 together forming an essentially planar sensing surface;
a flow distributor 201, comprising a flow inlet 203', a flow channel 208 and a flow outlet 203" in order to establish a liquid flow of analytes along the sensing surface;
wherein the sensor being **characterized in that**:
the flow distributor comprises a ceiling 206 having a surface facing said flow channel, where said ceiling surface is more hydrophobic than said sensing surface
the flow inlet and the flow outlet are located in the plane of said ceiling surface, where the plane of said ceiling surface is essentially parallel to that of the sensing surface, and **in that**
the array of electrodes (214-224), is arranged so that, in the direction from the flow inlet to the flow outlet, the array of electrodes consecutively comprises a first blank electrode, at least one measuring electrode, a second blank electrode, at least one measuring electrode and optionally a third blank electrode.

2. A sensor according to claim 1, wherein the at least one measuring electrode comprises an enzyme capable of enzymatically converting the selected analyte and thereby providing a signal representative of the concentration of said analyte.

3. A sensor according to claim 1 or 2, wherein the at least one measuring electrode is a glucose electrode or a lactate electrode or a pyruvate electrode.

4. A sensor according to claim 3, consecutively comprising a first blank electrode, a first glucose electrode, a second blank electrode, a second glucose electrode and optionally third blank and glucose electrodes.

5. A sensor according to claim 1 or 2, wherein the at least one measuring electrode alternatively is
a glucose electrode and a lactate electrode, or
a lactate electrode and a pyruvate electrode, or
a glucose and pyruvate electrode.

6. A sensor according to claim 1 or 2, wherein the at least one measuring electrode is a glucose electrode, a lactate electrode and pyruvate electrode.

7. A sensor according to claim 1 or 2, wherein the array of electrodes is arranged so that, in the direction from the flow inlet to the flow outlet, it either consecutively comprises a first blank electrode, a first lactate electrode, a first glucose electrode, a second blank electrode, a second lactate electrode and a second glucose electrode, or consecutively comprises a first blank electrode a first glucose electrode, a first lactate electrode, a second blank electrode, a second glucose electrode and a second lactate electrode.

8. A sensor according to claim 7, further comprising a first pyruvate electrode located between the first blank and the second blank electrode, and a second pyruvate electrode located after the second blank electrode.

9. A sensor according to claim 3, comprising at least two glucose or lactate or pyruvate electrode, wherein said at least two electrodes have different intervals of linear sensitivity.

10. A sensor according to claim 4 o 9, wherein the array of electrodes consecutively comprises a blank electrode, a first glucose electrode having a first interval of linear sensitivity , a second blank electrode, a second glucose electrode having a second interval of linear sensitivity, a third blank electrode and a third glucose electrode having a third interval of linear sensitivity.

11. A sensor according to claim 10, wherein the first interval of linear sensitivity relates to the highest concentration of glucose.

12. A sensor according to claim 11, wherein the first interval of linear sensitivity corresponds to glucose concentrations between about 5 to 100 mM, the second interval of linear sensitivity corresponds to glucose concentrations between about 1 to 25 mM, and the third interval of linear sensitivity corresponds to glucose concentrations between about 0.1 to 5 mM.

13. A sensor according to any preceding claim, wherein the array of electrodes further comprises a reference electrode and/or a counter electrode, preferably located after the measuring and blank electrodes.

14. A sensor according to claim 1, comprising an upper part extending over the sensing surface and being provided with a flow inlet and a flow outlet for establishing a liquid flow of analytes in the flow channel along the sensing surface.

15. A sensor according to any preceding claim, wherein the at least one measuring electrode includes multiple membrane layers, comprising
an oxidase membrane layer comprising immobilized oxidase enzyme capable of reacting the analyte with oxygen in a hydrogen peroxide generating reaction; and
a diffusion limiting membrane adapted to provide a higher diffusion resistance for the analyte than for oxygen and to provide lower flow of analyte to the oxidase membrane layer than the conversion rate of the oxidase enzyme.

## Patentansprüche

1. Sensor zum kontinuierlichen Nachweis von einem oder mehreren Analyten in einem Flüssigkeitsstrom, umfassend:
einen Elektrodenteil 204, umfassend ein Array von Elektroden 214-224, die zusammen eine im Wesentlichen ebene Sensorfläche bilden;
einen Strömungsverteiler 201, umfassend einen Stromeinlass 203', einen Strömungskanal 208 und einen Stromauslass 203", um einen Flüssigkeitsstrom des Analyten entlang der Sensorfläche herzustellen;
wobei der Sensor **dadurch gekennzeichnet ist, dass**:
der Strömungsverteiler eine Decke 206 mit einer Oberfläche umfasst, die zu dem Strömungskanal weist, wobei die Decken-Oberfläche hydrophober als die Sensorfläche ist
der Stromeinlass und der Stromauslass in der Ebene der Decken-Oberfläche angeordnet sind, wobei die Ebene der Decken-Oberfläche im Wesentlichen parallel zu jener der Sensorfläche ist, und dass
das Array der Elektroden (214-224) so angeordnet ist, dass in der Richtung von dem Stromeinlass zu dem Stromauslass das Array der Elektroden in Folge eine erste Vergleichs-Elektrode, mindestens eine Mess-Elektrode, eine zweite Vergleichs-Elektrode, mindestens eine Mess-Elektrode und gegebenenfalls eine dritte Vergleichs-Elektrode umfasst.

2. Sensor nach Anspruch 1, wobei die mindestens eine Mess-Elektrode ein Enzym umfasst, das den ausgewählten Analyten enzymatisch umwandeln kann und dabei ein für die Konzentration des Analyten repräsentatives Signal bereitstellt.

3. Sensor nach Anspruch 1 oder 2, wobei die mindestens eine Mess-Elektrode eine Glucose-Elektrode oder eine Lactat-Elektrode oder eine Pyruvat-Elektrode ist.

4. Sensor nach Anspruch 3, in Folge umfassend eine erste Vergleichs-Elektrode, eine erste Glucose-Elektrode, eine zweite Vergleichs-Elektrode, eine zweite Glucose-Elektrode und gegebenenfalls dritte Vergleichs- und Glucose-Elektroden.

5. Sensor nach Anspruch 1 oder 2, wobei alternativ die mindestens eine Mess-Elektrode
eine Glucose-Elektrode und eine Lactat-Elektrode, oder
eine Lactat-Elektrode und eine Pyruvat-Elektrode, oder
eine Glucose- und Pyruvat-Elektrode ist.

6. Sensor nach Anspruch 1 oder 2, wobei die mindestens eine Mess-Elektrode eine Glucose-Elektrode, eine Lactat-Elektrode und Pyruvat-Elektrode ist.

7. Sensor nach Anspruch 1 oder 2, wobei das Array von Elektroden so angeordnet ist, dass in der Richtung von dem Stromeinlass zu dem Stromauslass, es entweder in Folge eine erste Vergleichs-Elektrode, eine erste Lactat-Elektrode, eine erste Glucose-Elektrode, eine zweite Vergleichs-Elektrode, eine zweite Lactat-Elektrode und eine zweite Glucose-Elektrode umfasst, oder in Folge eine erste Vergleichs-Elektrode eine erste Glucose-Elektrode, eine erste Lactat-Elektrode, eine zweite Vergleichs-Elektrode, eine zweite Glucose-Elektrode und eine zweite Lactat-Elektrode umfasst.

8. Sensor nach Anspruch 7, weiterhin umfassend eine erste Pyruvat-Elektrode, angeordnet zwischen der ersten Vergleichs- und der zweiten Vergleichs-Elektrode, und eine zweite Pyruvat-Elektrode, angeordnet nach der zweiten Vergleichs-Elektrode.

9. Sensor nach Anspruch 3, umfassend mindestens zwei Glucose- oder Lactat- oder Pyruvat-Elektrode, wobei die mindestens zwei Elektroden unterschiedliche Intervalle von linearer Empfindlichkeit aufweisen.

10. Sensor nach Anspruch 4 o 9, wobei die Array von Elektroden in Folge eine Vergleichs-Elektrode, eine erste Glucose-Elektrode mit einem ersten Intervall von linearer Empfindlichkeit, eine zweite Vergleichs-Elektrode, eine zweite Glucose-Elektrode mit einem zweiten Intervall von linearer Empfindlichkeit, eine dritte Vergleichs-Elektrode und eine dritte Glucose-Elektrode mit einem dritten Intervall von linearer Empfindlichkeit umfasst.

11. Sensor nach Anspruch 10, wobei das erste Intervall von linearer Empfindlichkeit sich auf die höchste Konzentration von Glucose bezieht.

12. Sensor nach Anspruch 11, wobei das erste Intervall von linearer Empfindlichkeit Glucose-Konzentrationen zwischen etwa 5 bis 100 mM entspricht, das zweite Intervall von linearer Empfindlichkeit Glucose-Konzentrationen zwischen etwa 1 bis 25 mM entspricht und das dritte Intervall von linearer Empfindlichkeit Glucose-Konzentrationen zwischen etwa 0,1 bis 5 mM entspricht.

13. Sensor nach einem vorangehenden Anspruch, wobei das Array von Elektroden weiterhin eine Bezugs-Elektrode und/oder eine Gegen-Elektrode, vorzugsweise angeordnet nach den Mess- und Vergleichs-Elektroden, umfasst.

14. Sensor nach Anspruch 1, umfassend einen oberen Teil, der sich über die Sensorfläche erstreckt und versehen mit einem Stromeinlass und einem Stromauslass zum Herstellen eines Flüssigkeitsstroms von Analyten in dem Strömungskanal entlang der Sensorfläche.

15. Sensor nach einem vorangehenden Anspruch, wobei die mindestens eine Mess-Elektrode Mehrfachmembran-Schichten einschließt, umfassend
eine Oxidase-Membran-Schicht, umfassend immobilisiertes Oxidase-Enzym, das mit dem Analyten mit Sauerstoff in einer Wasserstoffperoxid erzeugenden Reaktion reagieren kann; und
eine Diffusions-begrenzende Membran, angepasst zum Bereitstellen eines höheren Diffusionswiderstands für den Analyten als für Sauerstoff und zum Bereitstellen von geringerer Strömung von Analyten zu der Oxidase-Membran-Schicht als die Umwandlungsrate des Oxidase-Enzyms.

## Revendications

1. Capteur pour la détection en continu d'un ou plusieurs analytes dans un flux liquide, comprenant :
une partie d'électrode 204 comprenant un réseau d'électrodes 214-224 formant ensemble une surface de capteur essentiellement plane ;
un distributeur de flux 201, comprenant une entrée de flux 203', un canal de flux 208 et une sortie de flux 203" afin d'établir un flux liquide d'analytes le long de la surface de capteur ;
dans lequel le capteur est **caractérisé en ce que :**
le distributeur de flux comprend un plafond 206 ayant une surface faisant face audit canal de flux, où ladite surface de plafond est plus hydrophobe que ladite surface de capteur
l'entrée de flux et la sortie de flux sont localisées dans le plan de ladite surface de plafond, où le plan de ladite surface de plafond est essentiellement parallèle à celle de la surface de capteur, et **en ce que**
le réseau d'électrodes (214-224), est arrangé de sorte que, dans la direction de l'entrée de flux vers la sortie de flux, le réseau d'électrodes comprend consécutivement une première électrode vierge, au moins une électrode de mesure, un deuxième électrode vierge, au moins une électrode de mesure et facultativement une troisième électrode vierge.

2. Capteur selon la revendication 1, dans lequel la au moins une électrode de mesure comprend une enzyme capable de convertir enzymatiquement l'analyte sélectionné et ainsi fournir un signal représentatif de la concentration de l'analyte.

3. Capteur selon la revendication 1 ou 2, dans lequel la au moins une électrode de mesure est une électrode à glucose ou une électrode à lactate ou une électrode à pyruvate.

4. Capteur selon la revendication 3, comprenant consécutivement une première électrode vierge, une première électrode à glucose, une deuxième électrode vierge, une deuxième électrode à glucose et facultativement des troisièmes électrodes vierges et à glucose.

5. Capteur selon la revendication 1 ou 2, dans lequel la au moins une électrode de mesure est alternativement
une électrode à glucose et une électrode à lactate, ou
une électrode à lactate et une électrode à pyruvate, ou
une électrode à glucose et à pyruvate.

6. Capteur selon la revendication 1 ou 2, dans lequel la au moins une électrode de mesure est une électrode à glucose, une électrode à lactate et une électrode à pyruvate.

7. Capteur selon la revendication 1 ou 2, dans lequel le réseau d'électrodes est arrangé de sorte que, dans la direction de l'entrée de flux vers la sortie de flux, soit il comprend consécutivement une première électrode vierge, une première électrode à lactate, une première électrode à glucose, une deuxième électrode vierge, une deuxième électrode à lactate et une deuxième électrode à glucose, soit il comprend consécutivement une première électrode vierge une première électrode à glucose, une première électrode à lactate, une deuxième électrode vierge, une deuxième électrode à glucose et une deuxième électrode à lactate.

8. Capteur selon la revendication 7, comprenant en outre une première électrode à pyruvate localisée entre la première et la deuxième électrode vierge, et une deuxième électrode à pyruvate localisée après la deuxième électrode vierge.

9. Capteur selon la revendication 3, comprenant au moins deux électrodes à glucose ou à lactate ou à pyruvate, dans lequel lesdites au moins deux électrodes ont des intervalles de sensibilité linéaire différents.

10. Capteur selon la revendication 4 o 9, dans lequel le réseau d'électrodes comprend consécutivement une électrode vierge, une première électrode à glucose ayant un premier intervalle de sensibilité linéaire, une deuxième électrode vierge, une deuxième électrode à glucose ayant un deuxième intervalle de sensibilité linéaire, une troisième électrode vierge et une troisième électrode à glucose ayant un troisième intervalle de sensibilité linéaire.

11. Capteur selon la revendication 10, dans lequel le premier intervalle de sensibilité linéaire concerne la concentration en glucose la plus élevée.

12. Capteur selon la revendication 11, dans lequel le premier intervalle de sensibilité linéaire correspond à des concentrations en glucose entre environ 5 à 100 mM, le deuxième intervalle de sensibilité linéaire correspond à des concentrations en glucose entre environ 1 à 25 mM, et le troisième intervalle de sensibilité linéaire correspond à des concentrations en glucose entre environ 0,1 à 5 mM.

13. Capteur selon l'une quelconque des revendications précédentes, dans lequel le réseau d'électrodes comprend en outre une électrode de référence et/ou une contre électrode, préférablement localisée après les électrodes de mesure et vierges.

14. Capteur selon la revendication 1, comprenant une partie supérieure s'étendant par-dessus la surface de capteur et étant pourvue d'une entrée de flux et d'une sortie de flux pour établir un flux liquide d'analytes dans le canal de flux le long de la surface de capteur.

15. Capteur selon l'une quelconque des revendications précédentes, dans lequel la au moins une électrode de mesure inclut de couches de membrane multiples, comprenant
une couche de membrane oxydase comprenant une enzyme oxydase immobilisée capable de faire réagir l'analyte avec de l'oxygène dans une réaction produisant du peroxyde d'hydrogène ; et
une membrane limitant la diffusion adaptée pour fournir une résistance de diffusion plus importante pour l'analyte que pour l'oxygène et pour fournir un flux d'analytes plus bas à la couche de membrane oxydase que le taux de conversion de l'enzyme oxydase.
